# EUROPEAN PATENT SPECIFICATION

(11) **EP 1 299 520 B1**
(45) Date of publication and mention of the grant of the patent: **12.09.2007**
(21) Application number: 01957121.5
(22) Date of filing: 11.07.2001
(51) Int. Cl.: C11D 17/04, C11D 1/83, C11D 3/50, C11D 3/22, A61L 9/05

(54) **LAVATORY FRESHENING AND/OR CLEANING SYSTEM AND METHOD**
ERFRISCHUNGS- UND/ODER REINIGUNGSSYSTEM FÜR TOILETTEN UND VERFAHREN
SYSTEME ET PROCEDE DE RAFRAICHISSEMENT ET/OU DE NETTOYAGE DES TOILETTES

(30) Priority: 12.07.2000 GB 0017151
(43) Date of publication of application: 09.04.2003
(73) Proprietor: S. C. Johnson & Son, Inc., Racine, WI 53403 (US)
(72) Inventor: MOODYCLIFFE, Timothy, I., Milwaukee, WI 53202 (US); VELTMAN, Jerome, J., Racine, WI 53402 (US)
(74) Representative: Carpmaels & Ransford
(86) International application number: PCT/US2001/021789
(87) International publication number: WO 2002/004592

(56) References cited:
- WO-A-99/66139
- CA-A- 2 201 406
- DE-A- 19 918 183
- US-A- 6 028 045
- US-A- 6 066 293

## Description

This invention relates to a freshener and/or cleaner system for the lavatory and to a method of using such a system in a lavatory bowl. In particular, this invention relates to a system comprising a liquid freshening and/or cleaning composition and a liquid dispenser.

Several lavatory freshening and/or cleaning systems are known. These systems include "solid block" type systems, where a freshening and/or cleaning block is placed either under the rim of the lavatory or in the cistern. In the case of the under the rim system, water dissolves part of the block each time the lavatory is flushed, allowing the lavatory bowl to be cleaned and/or freshened. In the case of the cistern block system, part of the block dissolves in the cistern prior to flushing and the toilet is cleaned and freshened on flushing of the water held in the cistern into the lavatory bowl. However, solid toilet blocks have demonstrated several drawbacks, in particular their inability to deliver constant amounts of cleaning and freshening agents during the lifetime of the block.

Other freshening and cleaning systems are of the liquid-dispensing type. Such systems include a liquid dispenser and a liquid freshening and/or cleaning composition. A liquid dispenser suitable for such a system is disclosed in the applicant's published international patent application WO 99/66139. Such liquid dispensers generally comprise a reservoir and a liquid-conveying device in the form of a pad, or a plate having capillary channels formed therein, the liquid-conveying device and the reservoir being so connected as to allow the freshening and/or cleaning composition held in the reservoir to be transferred to the liquid-conveying device in a controlled manner. The liquid dispenser is positioned under the rim of a lavatory such that, during flushing, a sufficient amount of freshening and/or cleaning composition is transferred to the lavatory bowl to effect the cleaning of the bowl.

In order for liquid cleaning systems to be effective, the liquid dispenser must be provided with a suitable liquid cleaning and/or freshening composition. It is desirable that such a liquid composition possess certain properties in order to carry out its freshening and/or cleaning functions. In particular, it is desirable that, when the composition is dispensed by flushing, sufficient foaming occurs. Foaming is desirable in order to promote cleaning of the lavatory bowl and dispersal of any perfume which is contained in the composition. Foaming also confers certain aesthetic properties when the toilet is flushed.

A known liquid cleaning composition, stated to be suitable for liquid cleaning systems of the type hereinbefore described, is disclosed in European patent application EP-A-0 775 741, which describes a composition having a viscosity at room temperature of 10 to 2000 mPa s and comprising:
(a) 1 to 25 wt.% of perfume,
(b) 10 to 50 wt.% of anionic or non-ionic surfactant,
(c) 1 to 20 wt.% of non-evaporating, water soluble evaporation regulator, and
(d) balance solvent.

It would be desirable to provide alternative formulations, and particularly formulations which can be manufactured at lower cost.

Prior-art liquid compositions, and indeed also prior-art solid rim-blocks, typically comprise a surfactant level of at least 10 wt.%. This is believed to be because such a quantity would be required to generate the desired level of foam.

It has surprisingly been found by the inventors of the present invention that high levels of foam can, however, be achieved with compositions containing low levels of surfactant and which are also suitable for use in liquid dispensers of the above type. Additionally, it has been discovered that high levels of foam can be achieved with compositions containing low levels of surfactant while also dissolving, or micro-emulsifying, any perfume present in the liquid composition for freshening.

Thus, in accordance with the present invention, there is provided a lavatory freshening and/or cleaning system as defined in claim 1.

Preferably, the total surfactant concentration is within the range 2.5 to 8.0 wt.%, the most preferred value being substantially 7.6 wt.%.

Suitable surfactants are anionic and/or non-ionic surfactants, although a combination of anionic and non-ionic surfactants is particularly desirable. The preferred anionic surfactant is an alkyl ether sulphate, such as that marketed under the trade name Steol CS 270 which contains active surfactant at a concentration of 70 wt.%, and the preferred non-ionic surfactant is an ethoxylated synthetic alcohol, such as that marketed under the trade name Lutensol AO8.

Optionally, perfume may be present to provide freshening of the lavatory bowl and its vicinity. A suitable perfume for the liquid composition is that marketed under the trade name Vertana 114.737.

The preferred total concentration of perfume is within the range 4 to 15 wt.%, the most preferred value being substantially 6 wt.%.

Although the combination of surfactant and perfume can act as a thickening agent, the composition includes one or more additional thickening agents, having a preferred total concentration within the range 0.2 to 5 wt.%. The most preferred concentration of additional thickening agent is substantially 0.40 wt.%. A suitable thickening agent is a hydroxyethylcellulose such as that marketed under the trade name Natrasol 250 HHR.

In addition, humectants may also be present in the liquid composition. Humectants are desirable when a perfume is present, in order to regulate the evaporation of the perfume from the composition. Additionally, humectants are useful in preventing phase separation of, and precipitation from, the composition. Suitable humectants include glycols, glycoethers, alcohols, sugars and polyethers.

Optionally, the composition may comprise sequestrants, pH control agents, dyes and preservatives.

The invention extends to a method of use of such a lavatory freshening and/or cleaning system in a lavatory bowl.

A preferred embodiment of the present invention incorporates a liquid composition having the following components:

| **Weight percent** | **Common name** | **Chemical name** | **Component type** | **Function** |
|---|---|---|---|---|
| | | | | |
| | Water | Water | | Solvent |
| 7.25 | Steol CS 270 (Containing 70% active surfactant) | Sodium Lauryl Ether Sulphate | Anionic surfactant | Perfume solubilisation, foam generation and viscosity building |
| 2.50 | Lutensol AO8 | | Non-ionic surfactant | Perfume solubilisation, foam generation and viscosity building |
| 7.00 | | Dipropylene Glycol | Short-chain hydrocarbon | Humectant |
| 2.00 | Dequest 2010 | | Phosphonate | Sequestrant |
| 1.60 | | Sodium Hydroxide (32 wt.% aqueous solution) | | Control of pH |
| 0.40 | Natrasol 250 HHR | | Cellulosic | Thickener |
| 0.005 | | | | Dye |
| 6.00 | | | | Perfume |
| 0.02 | Myacide BT | | | Preservative |

As can be seen from the above table, the composition comprises Steol CS 270. This includes an anionic surfactant at a concentration of 70 wt.%, so that the actual concentration of anionic surfactant in the composition is 5.1 wt.%, resulting in a total surfactant concentration in the composition of 7.6 wt.%.

## Claims

1. A lavatory freshening and/or cleaning system comprising a dispenser arranged to be positioned under the rim of a lavatory bowl for dispensing a liquid composition therefrom, such that, during flushing, a sufficient amount of the liquid composition is transferred to the lavatory bowl to effect the cleaning thereof;
**characterised in that** said system further comprises a liquid composition wherein said liquid composition comprises a thickening agent and one or more surfactants wherein the total concentration of surfactants does not exceed 8 wt,%.

2. A system as claimed in claim 1, wherein the total surfactant concentration is within the range 2.5 to 8.0 wt.%.

3. A system as claimed in claim 2, wherein the total surfactant concentration is substantially 7.6 wt.%.

4. A system as claimed in any preceding claim, wherein said surfactant is either an anionic surfactant or a non-ionic surfactant or a combination thereof.

5. A system as claimed in claim 4, comprising sodium lauryl ether sulphate (Steol CS 270; Registered Trade Mark) constituting a least one anionic surfactant.

6. A system as claimed in claim 4 or claim 5, comprising PEG-8 straight chain C13-15 fatty alcohol (Lutensol AO8; Registered Trade Mark) constituting at least one non-ionic surfactant.

7. A system as claimed in claim 1, wherein the concentration of said thickening agent, apart from the surfactant and any perfume which may be present, is within the range 0.2 to 5 wt.%.

8. A system as claimed in claim 1 or claim 7, wherein said thickening agent is a hydroxyethylcellulose.

9. A system as claimed in any preceding claim, wherein said liquid composition further comprises perfume.

10. A system as claimed in claim 9, wherein the total perfume concentration is within the range 4 to 15 wt.%.

11. A system as claimed in claim 9 or claim 10, wherein the total perfume concentration is substantially 6 wt.%.

12. A system as claimed in any preceding claim, wherein said liquid composition further comprises one or more of sequestrants, pH control agents, dyes and preservatives.

13. A method of using in a lavatory bowl a lavatory freshening and/or cleaning system as claimed in any preceding claim.

## Patentansprüche

1. Toiletten-Auffrischungs- und/ oder Reinigungssystem mit einem Spender, der unter den Rand einer Toilettenschüssel positionierbar ausgeführt ist, um eine flüssige Zusammensetzung auszugeben derart, dass beim Spülen eine zum Reinigen der Toilettenschüssel ausreichende Menge der flüssigen Zusammensetzung in dieselbe ausgegeben wird, **dadurch gekennzeichnet, dass** das System weiterhin eine flüssige Zusammensetzung aufweist, die ein Eindickungsmittel sowie eine oder mehrere grenzflächenaktive Substanzen enthält, deren Konzentration insgesamt 8 Gew.-% nicht übersteigt.

2. System nach Anspruch 1, bei dem die Konzentration der grenzflächenaktiven Substanz insgesamt im Bereich von 2,5 Gew.-% bis 8 Gew.-% liegt.

3. System nach Anspruch 2, bei dem die Konzentration der grenzflächenaktiven Substanz insgesamt im wesentlichen 7,6 Gew.-% beträgt.

4. System nach einem der vorgehenden Ansprüche, bei dem die grenzflächenaktive Substanz entweder anionisch oder nichtionisch jeweils einzeln oder in Kombination ist.

5. System nach Anspruch 4, das als die mindestens eine anionische grenzflächenaktive Substanz Natriumlaurylethersulfat (Steol CS 270; eingetragenes Warenzeichen) aufweist.

6. System nach Anspruch 4 oder 5, das als die mindestens eine nichtionische grenzflächenaktive Substanz einen PEG-8 gradkettigen C13-15 Fettalkohol (Lutensol A08; eingetragenes Warenzeichen) aufweist.

7. System nach Anspruch 1, bei dem abgesehen von der grenzflächenaktiven Substanz und einem ggf. vorliegenden Duftstoff die Konzentration des Eindickungsmittels im Bereich von 0,2 Gew.-% bis 5 Gew.-% liegt.

8. System nach Anspruch 1 oder 7, bei dem das Eindickungsmittel eine Hydroxyethylcellulose ist.

9. System nach einem der vorgehenden Ansprüche, bei dem die flüssige Zusammensetzung weiterhin einen oder mehrere Duftstoffe aufweist.

10. System nach Anspruch 9, bei dem die Duftstoff-Gesamtkonzentration im Bereich von 4 Gew.-% bis 15 Gew.-% liegt.

11. System nach Anspruch 9 oder 10, bei dem die Duftstoff-Gesamtkonzen- , tration im wesentlichen 6 Gew.-% beträgt.

12. System nach einem der vorgehenden Ansprüche, bei dem die flüssige Zusammensetzung weiterhin einen oder mehr Inhaltsstoffe aus der Gruppe Sequestrierungsmittel, pH-Einsteller, Farbstoffe und Konservierungsmittel enthält.

13. Verfahren der Anwendung eines Auffrischungs- und /oder Reinigungssystems nach einem der vorgehenden Ansprüche in einer Toilettenschüssel.

## Revendications

1. Système pour rafraîchir et/ou nettoyer des toilettes comprenant un distributeur agencé pour être positionné sous le rebord d'une cuvette de toilettes pour distribuer une composition liquide à partir de celui-ci, de telle sorte que, pendant le rinçage, une quantité suffisante de la composition liquide soit transférée vers la cuvette des toilettes afin de réaliser son nettoyage ;
**caractérisé en ce que** ledit système comprend de plus une composition liquide dans lequel ladite composition liquide comprend un agent épaississant et un ou plusieurs tensio-actifs, dans lequel la concentration totale des tensio-actifs n'excède pas 8% en poids.

2. Système selon la revendication 1, dans lequel la concentration totale en tensio-actifs se trouve dans l'intervalle de 2.5 à 8.0% en poids.

3. Système selon la revendication 2, dans lequel la concentration totale en tensio-actifs est pratiquement de 7,6% en poids.

4. Système selon l'une quelconque des revendications précédentes, dans lequel ledit tensio-actif est soit un tensio-actif anionique soit un tensio-actif non-ionique soit une combinaison de ceux-ci.

5. Système selon la revendication 4 comprenant du sulfate de sodium lauryléther (Steol CS 270 ; marque déposée) constituant au moins un tensio-actif anionique.

6. Système selon la revendication 4 ou la revendication 5 comprenant un alcool gras en C₁₃-C₁₅ linéaire de PEG-8 (Lutensol AO8 ; marque déposée) constituant au moins un tensio-actif non-ionique.

7. Système selon la revendication 1, dans lequel la concentration dudit agent épaississant, à part le tensio-actif et tout parfum qui peut être présent, se trouve dans l'intervalle de 0.2 à 5% en poids.

8. Système selon la revendication 1 ou la revendication 7, dans lequel ledit épaississant est une hydroxycellulose.

9. Système selon l'une quelconque des revendications précédentes, dans lequel ladite composition liquide comprend de plus du parfum.

10. Système selon la revendication 9, dans lequel la concentration totale en parfum se trouve dans l'intervalle de 4 à 15% en poids.

11. Système selon la revendication 9 ou la revendication 10, dans lequel la concentration totale en parfum est pratiquement de 6% en poids.

12. Système selon l'une quelconque des revendications précédentes, dans lequel ladite composition liquide comprend de plus un ou plusieurs agents séquestrants, agents de contrôle du pH, colorants et conservateurs.

13. Procédé pour l'utilisation dans une cuvette de toilettes d'un système pour rafraîchir et /ou nettoyer des toilettes selon l'une quelconque des revendications précédentes.
